# EUROPÄISCHE PATENTSCHRIFT

(11) **EP 2 745 866 B1**
(45) Veröffentlichungstag und Bekanntmachung des Hinweises auf die Patenterteilung: **05.10.2016**
(21) Anmeldenummer: 14153335.6
(22) Anmeldetag: 30.01.2014
(51) Int. Cl.: A61M 5/20, A61M 5/32

(54) **AUSLÖSESICHERUNG FÜR EINEN AUTOINJEKTOR**
Release safety device for an auto-injector
Sécurité de déclenchement pour un injecteur de voiture

(43) Veröffentlichungstag der Anmeldung: 25.06.2014
(73) Patentinhaber: TecPharma Licensing AG, 3401 Burgdorf (CH)
(72) Erfinder: Hirschel, Jürg, 3007 Bern (CH); Tschirren, Markus, 3400 Burgdorf (CH)
(74) Vertreter: Schwabe - Sandmair - Marx

(56) Entgegenhaltungen:
- WO-A1-2008/113864
- WO-A1-2009/155277
- WO-A1-2010/136078
- WO-A1-2011/012849
- GB-A- 2 467 904
- US-A1- 2013 018 313

## Beschreibung

Die Erfindung betrifft eine Auslösesicherung für einen Autoinjektor, um eine ungewollte Produktausschüttung zu verhindern. Der Autoinjektor dient zur Ausschüttung eines flüssigen Produkts, insbesondere eines Medikaments.

Ein Autoinjektor ist eine Injektionsvorrichtung, mittels der ein in einem Produktbehälter enthaltenes flüssiges Produkt mittels eines Antriebsmechanismus, wie zum Beispiel einer vorgespannten Feder oder eines Gasgenerators, ausgeschüttet und einem Patienten verabreicht werden kann.

Autoinjektoren sind im Stand der Technik bekannt. Zum Beispiel wird in der EP 2 742 962 A2 ein Autoinjektor beschrieben, der eine in dem Gehäuse feststehende Spritze aufweist. Zum Verabreichen wird eine als Auslöseglied dienende Nadelschutzhülse an die gewünschte Einstichstelle des Patienten angesetzt. Indem der Autoinjektor gegen die Einstichstelle gedrückt wird, wird die Nadelschutzhülse in das Gehäuse zurückgeschoben, wobei einerseits die Nadel der Spritze in den Patienten eingestochen und anderseits der Antriebsmechanismus aktiviert wird, wodurch das in dem Produktbehälter enthaltene Produkt von einem Vortriebsglied durch die Nadel ausgestoßen wird.

Aus der WO 2008/113199 A1 ist ein Autoinjektor bekannt, bei dem die Nadelschutzhülse ebenfalls an die gewünschte Einstichstelle angesetzt und durch Andrücken des Autoinjektors an die Einstichstelle in das Gehäuse zurückgeschoben wird. Hierdurch wird eine Einstichsequenz ausgelöst, mit der der Produktbehälter relativ zu dem Gehäuse soweit in die distale Richtung verschoben wird, dass die Nadel über das distale Ende der Nadelschutzhülse hervortritt und in den Patienten eingestochen wird. Gegen Ende der Einstichsequenz wird eine Ausschüttsequenz ausgelöst, bei der ein Vortriebsglied das in dem Produktbehälter enthaltene Produkt über die Nadel ausstößt und in den Patienten injiziert. Am Ende der Injektion wird der Autoinjektor von der Einstichstelle abgenommen, wodurch eine Rückziehsequenz gestartet wird, mittels der der Produktbehälter relativ zu dem Gehäuse in die proximale Richtung verschoben wird, wodurch die Nadel in das distale Ende des Autoinjektors eingezogen wird, um Verletzungen zu verhindern.

Aus der WO 2010/136078 A1 ist ein Autoinjektor bekannt, dessen Nadelschutzhülse ebenfalls an die gewünschte Einstechstelle angesetzt und durch Andrücken des Autoinjektors an die Einstechstelle in das Gehäuse zurückgeschoben wird. Wie bei der WO 2008/113199 A1 wird auch hier eine Einstechsequenz ausgelöst, mit der der Produktbehälter relativ zu dem Gehäuse zum Einstechen der Nadel in die distale Richtung verschoben wird. Ein versehentliches Auslösen der Einstechsequenz wird dadurch verhindert, dass die Abdeckkappe die Verschiebung der Nadelschutzhülse blockiert.

Außerdem gibt es Autoinjektoren, die in einer gleichen Weise wie im oben genannten Stand der Technik, d. h. durch Verschieben einer Nadelschutzhülse in das Gehäuse ausgelöst werden, wobei der Produktbehälter nur zum Einstechen relativ zu dem Gehäuse verschiebbar ist, wobei die Nadel nach dem Einstechen in der hervorgetretenen Position verbleibt. Zum Schutz der Nadel wird die Nadelschutzhülse über das distale Ende der Nadel geschoben und insbesondere verriegelt, um ein erneutes Zurückschieben der Nadelschutzhülse in das Gehäuse zu verhindern.

Durch das Konzept, das Einstechen bzw. das Ausschütten durch Zurückschieben des Auslöseglieds, welches oftmals eine Nadelschutzhülse ist, in das Gehäuse auszulösen, besteht die Gefahr, dass eine Produktausschüttung versehentlich ausgelöst werden kann, zum Beispiel wenn der Autoinjektor herunterfällt oder anderweitig stark erschüttert wird. Dabei kann das Auslöseglied durch seine Massenträgheit relativ zu dem Gehäuse in seine Auslöseposition verschoben werden, wodurch die Produktausschüttung unbeabsichtigt ausgelöst wird.

Die WO 2009/114542 A1 schlägt ein Sicherungsmittel vor, welches an dem der Nadel gegenüberliegenden Ende, d. h. dem proximalen Ende, angebracht ist und vor dem Auslösen des Autoinjektors entfernt werden muss. Das Sicherungsmittel blockiert die Bewegung der Auslösehülse in seine Auslöseposition. Zum Vorbereiten des Autoinjektors für die Ausschüttung wird eine am distalen Ende des Autoinjektors angebrachte Abdeckkappe abgenommen und das am proximalen Ende befestigte Sicherungsmittel entfernt. Durch die zwei getrennt voneinander vorzunehmenden Schritte für die Vorbereitung des Autoinjektors wird eine intuitive Handhabung erschwert.

Weitere Autoinjektoren sind aus der US 2004/0039336 A1 und der WO 94/11041 A1 bekannt.

Es ist eine Aufgabe der Erfindung, einen Autoinjektor bereitzustellen, der eine versehentliche Auslösung der Einstech- oder Ausschüttsequenz verhindert und intuitiv handhabbar ist.

Die Aufgabe wird von dem Autoinjektor gemäß Anspruch 1 gelöst. Vorteilhafte Weiterentwicklungen ergeben sich aus den abhängigen Ansprüchen, der Beschreibung und den Figuren.

Die Erfindung geht von einem Autoinjektor zur Ausschüttung eines flüssigen Produkts, insbesondere eines Medikaments, aus. Der Autoinjektor umfasst ein Gehäuse, welches vorzugsweise länglich und/oder hülsenförmig ist. Das Gehäuse dient dazu, von dem Verwender der Vorrichtung mit einer Hand umgriffen zu werden. In dem Gehäuse ist ein Produktbehälter angeordnet. Bei dem Produktbehälter kann es sich insbesondere um eine Spritze handeln, welche einen Spritzenkörper aufweist, an dessen distalem Ende eine Injektionsnadel fest angeordnet ist. Der vorzugsweise zylindrische Spritzenkörper umgibt einen Kolben, der in Bezug auf den Spritzenkörper verschiebbar ist und für die Produktausschüttung zu dem distalen Ende hin verschoben wird, wodurch das zwischen dem Kolben und der Injektionsnadel angeordnete flüssige Produkt, insbesondere Medikament, durch die Injektionsnadel aus dem Produktbehälter ausgeschüttet wird. Der Spritzenkörper kann an seinem proximalen, d. h. hinteren oder der Injektionsnadel gegengesetzten Ende einen Flansch, der auch als Fingerflansch bezeichnet werden kann, aufweisen. Eine derartig ausgebildete Spritze ist als Standardspritze erhältlich, so dass für den Autoinjektor nicht zwingend eine speziell angepasste Spritze entwickelt werden braucht. Der Kolben liegt dichtend an dem Innendurchmesser des Spritzenkörpers an.

Das vorzugsweise längliche, hülsenförmige Gehäuse bildet die Längsachse des Autoinjektors. Das Gehäuse ist vorzugsweise zylindrisch, insbesondere kreiszylindrisch. Der Produktbehälter ist in dem Gehäuse angeordnet. Beispielsweise kann der Behälter verschiebbar in dem Gehäuse angeordnet sein, d. h. für ein automatisches Einstechen relativ zu dem Gehäuse in distale Richtung verschiebbar sein, so dass die Nadelspitze aus einer Öffnung am distalen Ende des Autoinjektors hervortritt und automatisch in den Patienten eingestochen werden kann. Optional kann bei einer solchen Vorrichtung die Nadelspitze nach erfolgter Produktausschüttung in das distale Ende der Vorrichtung hineinbewegt werden, insbesondere der Produktbehälter relativ zu dem Gehäuse in die proximale Richtung bewegt werden.

In bevorzugten Ausführungen ist der Produktbehälter entlang der Längsachse unverschiebbar in dem Gehäuse aufgenommen, insbesondere mittels eines Produktbehälterhalters oder Spritzenhalters, der den Prodüktbehälter axialfest hält und axialfest mit dem Gehäuse verbunden, insbesondere verrastet ist. Bevorzugt ragt die Nadelspitze distal über das distale Ende des Gehäuses. Hierdurch kann die Nadel mittels einer Bewegung des Gehäuses zum Patienten hin in die Einstichstelle eingestochen werden.

Der Autoinjektor umfasst ein insbesondere hülsenförmiges Auslöseglied, welches in seiner Ausgangsposition über das distale Ende des Gehäuses ragt. Das Auslöseglied dient primär zur Auslösung der Produktausschüttung oder ggf. der Einstechsequenz. Optional kann das Auslöseglied als Nadelschutzhülse dienen und als solche bezeichnet werden. Das Auslöseglied, welches das distale Ende des Autoinjektors bildet, kann eine Öffnung für die Injektionsnadel aufweisen, wobei die Injektionsnadel durch die Öffnung hindurchtreten kann. Das Auslöseglied kann in seiner Ausgangsposition in Bezug auf die Nadelspitze so angeordnet sein, dass das Auslöseglied, insbesondere die Nadelschutzhülse, distal über die Nadelspitze steht oder dass die Nadelspitze distal über das distale Ende des Auslöseglieds steht. Das Auslöseglied ist relativ zu dem Gehäuse aus seiner Ausgangsposition in die proximale Richtung um einen Betätigungshub in eine betätigte Position, insbesondere Betätigungsposition, verschiebbar, insbesondere in das Gehäuse verschiebbar, so dass die Nadel aus dem distalen Ende bzw. durch die Öffnung des Auslöseglieds hervortritt oder weiter hervortritt. Bevorzugt kann das Auslöseglied um einen Nadelschutzhub aus der Auslöseposition relativ zum dem Gehäuse in die distale Richtung in eine Nadelschutzposition verschoben werden, in der das distale Ende des Auslöseglieds, insbesondere der Nadelschutzhülse, distal über die Nadelspitze steht, um nach erfolgter Verwendung der Vorrichtung bzw. nach erfolgter Produktausschüttung eine Verletzungsgefahr, die von einer freiliegenden Nadelspitze ausgehen würde, zu verringern.

Das Auslöseglied kann zum Beispiel gegen die Kraft einer Feder, die zum Beispiel als Rückstellfeder oder Nadelschutzfeder bezeichnet werden kann, in die proximale Richtung verschoben werden, wobei die Feder das Auslöseglied aus der Position, in der ein Sperrmittel die Bewegung des Auslöseglieds in die proximale Richtung sperrt, in die distale Richtung verschieben kann oder aus der Auslöseposition in die distale Richtung, z.B. bis in die Nadelschutzposition verschieben kann.

Der Autoinjektor kann zum Beispiel ein federnd angeordnetes Vemiegelungsglied aufweisen, welches das Auslöseglied in seiner Nadelschutzposition, insbesondere in Bezug auf das Gehäuse verriegelt und ein Zurückschieben des Auslöseglieds in die proximale Richtung oder in das Gehäuse blockiert. Das Verriegelungsglied verriegelt das Auslöseglied zumindest so, dass die Nadel nicht aus dem distalen Ende des Auslöseglieds hervortreten kann. Das Auslöseglied kann zum Bespiel aus der Nadelschutzposition nur soweit in die proximale Richtung verschoben werden, dass die Nadelspitze nicht aus dem distalen Ende des Auslöseglieds hervortritt.

Der Autoinjektor umfasst eine Abdeckkappe, welche am distalen Ende des Gehäuses angeordnet ist, insbesondere in dem Auslieferungszustand des Autoinjektors. Die Abdeckkappe ist von dem Gehäuse abnehmbar, insbesondere abziehbar oder abdrehbar, insbesondere abschraubbar. Zum Vorbereiten des Autoinjektors für die bestimmungsgemäße Verwendung wird die Abdeckkappe von dem Gehäuse abgenommen. Die Abdeckkappe ist vorzugsweise an dem Gehäuse befestigt und umgibt das distale Ende des Auslöseglieds und verhindert insbesondere einen Zugriff auf das distale Ende des Auslöseglieds. Wenn die Abdeckkappe von dem Gehäuse abgenommen ist, ist der Zugriff auf das Auslöseglied freigegeben, so dass es an die Einstichstelle angesetzt werden kann.

Das insbesondere hülsenförmige Auslöseglied, welches in seiner Ausgangsposition über das distale Ende des Gehäuses ragt, ist für die Auslösung der Ausschüttung relativ zum Gehäuse aus der Ausgangsposition in eine proximale Richtung in die Auslöseposition bewegbar, wenn die Abdeckkappe von dem Gehäuse abgenommen ist. Das Auslöseglied ist mit einem Antriebsmechanismus derart gekoppelt, dass der Antriebsmechanismus die Produktausschüttung oder ggf. die Einstichsequenz freigibt, wenn das Auslöseglied in seiner Auslöseposition ist oder die Auslöseposition erreicht.

Das Auslöseglied wird gegen die Bewegung in die Auslöseposition blockiert, wenn die Abdeckkappe am Gehäuse angeordnet ist. Beispielsweise kann das Auslöseglied gar nicht oder nur um einen Hub in die proximale Richtung bewegt werden, der geringer ist als der Auslösehub, d. h. der Hub, den das Auslöseglied bei der Bewegung aus der Ausgangsposition in die Auslöseposition ausführt.

Bevorzugt kann der Autoinjektor ein Vortriebsglied, das zumindest während der Produktausschüttung auf den Kolben wirkt, insbesondere an dem Kolben anliegt, und eine Ausschüttfeder, die auf das Vortriebsglied wirkt, wie zum Beispiel sich insbesondere mit ihrem distalen Ende an dem Vortriebsglied abstützt, aufweisen. Das Vortriebsglied kann zum Bespiel hülsenförmig sein und eine Schulter, die zum Beispiel im Bereich des distalen Endes des Vortriebsglieds angeordnet ist, bilden, an der sich das distale Ende der Ausschüttfeder abstützen kann. Die Ausschüttfeder kann vorzugsweise innerhalb des z. B hülsenförmigen Vortriebsglieds angeordnet sein. Die Ausschüttfeder ist vorzugsweise eine als Druckfeder wirkende Wendelfeder, die zum Bespiel aus Metall gebildet ist. Die Ausschüttfeder kann so stark vorgespannt sein, insbesondere im Auslieferungszustand des Autoinjektors, dass sie bzw. die in ihr gespeicherte Energie ausreicht, um das Produkt aus dem Produktbehälter durch Verschieben des Vortriebsglieds um einen Ausschütthub im Wesentlichen vorständig auszuschütten. Durch die Verschiebung des Vortriebsglieds um den Ausschütthub wird auch der Kolben verschoben. Sofern im Auslieferungszustand zwischen dem Kolben und dem Vortriebsglied ein Abstand besteht, ist der Ausschütthub des Kolbens kleiner als der Ausschütthub des Vortriebsglieds, was bevorzugt ist, da der Kolben bis zur Verwendung unbelastet bleibt, wodurch eine ungewollte vorzeitige Produktausschüttung vermieden wird. Grundsätzlich ist es aber auch möglich, dass das Vortriebsglied im Auslieferungszustand und nicht erst bei der Produktausschüttung an dem Kolben anliegt. Sofern im Auslieferungszustand das Vortriebsglied bereites an dem Kolben anliegt, entspricht der Ausschütthub des Kolbens dem Ausschütthub des Vortriebsglieds. Das proximale Ende der Ausschüttfeder kann sich an dem Gehäuse oder einem gehäusefesten Element, insbesondere auch einen nur zeitweise zu dem Gehäuse axialfesten Element abstützen.

Besonders vorteilhaft lässt sich die Erfindung bei dem Autoinjektor aus der EP 2 742 962 A2 oder der WO 2008/113199 A1 einsetzen.

Das Auslöseglied kann ein z. B. gelenkig oder vorzugsweise federnd angeordnetes Sperrmittel aufweisen oder bilden. Das Auslöseglied kann einen hülsenförmigen Hauptkörper aufweisen, an dem das Sperrmittel gelenkig oder federnd angeordnet, insbesondere einstückig mit dem Hauptkörper gebildet ist. Das Sperrmittel ist von der Abdeckkappe in eine Sperrposition, in der das Sperrmittel einem von dem Autoinjektor gebildeten Anschlag gegenüberliegt, insbesondere in einer Flucht mit dem Anschlag ist, auslenkbar. Das Sperrmittel ist in seiner Freigabeposition, die das Sperrmittel einnimmt, wenn die Abdeckkappe abgenommen ist, an dem Anschlag vorbei bewegbar, d. h. nicht in der Flucht mit dem Anschlag. Die Abdeckkappe kann somit einen Aktuator bilden, der das Sperrmittel in die Freigabeposition drückt oder bewegen kann, insbesondere relativ zu dem Hauptkörper, wenn die Abdeckkappe an dem Gehäuse angeordnet ist. Wenn die Abdeckkappe von dem Gehäuse abgenommen ist, fehlt dieser Aktuator, wodurch das Sperrmittel nicht mehr in seiner Sperrposition gedruckt wird oder werden kann. Vorteilhaft wird dadurch erreicht, dass der Verwender des Autoinjektors mit dem Entfernen der Abdeckkappe von dem Gehäuse gleichzeitig das Auslöseglied für die Bewegung aus der Ausgangsposition in die Auslöseposition entsperrt. Wenn die Abdeckkappe an dem Gehäuse angeordnet ist, ist das Auslöseglied gegen die Bewegung in die Auslöseposition blockiert oder gesperrt. Durch das Sperrmittel kann der Autoinjektor nicht mehr durch die Massenträgheit des Auslöseglieds bei starker Erschütterung ausgelöst werden.

Vorzugsweise ist das Sperrmittel so angeordnet, insbesondere einstückig mit dem Auslöseglied bzw. dessen Hauptkörper gebildet, dass es in seine Freigabeposition federt, wenn es unbelastet ist und aus seiner Freigabeposition gegen seine Federkraft auslenkbar ist.

Vorzugsweise bildet das Gehäuse oder ein gehäusefestes Element, wie zum Bespiel der Produktbehälterhalter oder Spritzenhalter, den Anschlag. Das von dem Auslöseglied gebildete Sperrmittel wird mit dem Auslöseglied bei der Bewegung entlang der Längsachse des Autoinjektors mitbewegt, d. h. insbesondere auch bei der Bewegung des Auslöseglieds in die proximale Richtung mitbewegt. Dabei stößt das Sperrmittel an den Anschlag an und verhindert dadurch, dass das Auslöseglied noch weiter in die proximale Richtung bis in seine Auslöseposition bewegt werden kann.

Die Abdeckkappe kann zum Beispiel eine Aktuatorfläche aufweisen, welche das Sperrmittel in seine Sperrposition drückt oder in seiner Sperrposition hält. Beispielsweise kann die Abdeckkappe eine Haltefläche aufweisen, an der das Sperrmittel anliegt, wenn es in seiner Sperrposition ist. Zum Beispiel kann die Haltefläche die Aktuatorfläche sein oder eine separate Fläche von der Aktuatorfläche sein.

Insbesondere kann das Sperrmittel, insbesondere eine Kontaktfläche des Sperrmittel, die an den Anschlag anschlagen kann, von dem Anschlag entlang der Längsachse beabstandet sein, wenn das Auslöseglied in seiner Ausgangsposition ist. Dadurch wird dem Auslöseglied zwar grundsätzlich erlaubt, aus seiner Ausgangsposition in die proximale Richtung bewegt zu werden, allerdings nicht bis in seine Auslöseposition. Dadurch wird einerseits erreicht, dass relativ große Herstellungstoleranzen ermöglicht werden und anderseits verhindert, dass sich das Sperrmittel an dem Anschlag verklemmt und sich nicht mehr in seine Freigabeposition bewegt, auch wenn die Abdeckkappe abgenommen wird.

Die Haltefläche oder die Aktuatorfläche kann so konfiguriert sein, dass sie das Sperrmittel in der Sperrposition hält, wenn das Auslöseglied in seiner Ausgangsposition ist. Das Sperrmittel kann z.B. an der Haltefläche anliegen und/oder entlanggleiten, wenn das Auslöseglied aus der Ausgangsposition gegen die Feder bzw. deren Federkraft in die proximale Richtung verschoben wird und/oder aus der Position, in der das Sperrmittel die Bewegung des Auslöseglieds in die proximale Richtung sperrt, von der Feder bzw. deren Federkraft in die distale Richtung z.B. zurück bis in die Ausgangsposition verschoben wird.

Alternativ kann die Haltefläche so konfiguriert sein, dass das zusammen mit dem Auslöseglied bewegbare Sperrmittel erst an der Haltefläche anliegt, wenn das Sperrmittel, insbesondere seine Kontaktfläche, zu dem Anschlag hin und das Auslöseglied aus der Ausgangsposition in die proximale Richtung verschoben wurden. Insbesondere kann auch in dieser Alternative das Auslöseglied aus der Ausgangsposition gegen die Feder bzw. deren Federkraft in die proximale Richtung verschoben werden und/oder aus der Position, in der das Sperrmittel die Bewegung des Auslöseglieds in die proximale Richtung sperrt, von der Feder bzw. deren Federkraft in die distale Richtung z.B. zurück bis in die Ausgangsposition verschoben werden. In der Ausgangsposition kann das Sperrmittel auch bei aufgesetzter Abdeckkappe in seiner Freigabeposition oder in einer von der Sperrposition verschiedenen Position sein. Dies hat den Vorteil, dass gewisse materialbedingte Nachteile von Kunststoff beseitigt werden können. Manche Kunststoffe haben nämlich den Nachteil, dass sie zur Retardation oder zum Kriechen neigen, d. h. zur plastischen Verformung unter Last. Ist das Sperrmittel nämlich bereits in der Ausgangsposition des Auslöseglieds aus seiner Freigabeposition in seine Sperrposition ausgelenkt, insbesondere gefedert, kann es passieren, dass bei langer Lagerung, insbesondere auch in Abhängigkeit von der Temperatur, eine plastische Verformung der federnden Anlenkung des Sperrmittels erfolgt, wodurch bewirkt wird, dass das Sperrmittel nicht mehr in seine Freigabeposition zurückfedert, wenn die Abdeckkappe vom Gehäuse abgenommen wird. In diesem Fall würde das Auslöseglied trotz abgenommener Abdeckkappe nicht mehr in seine Auslöseposition bewegt werden. Dies könnte auch der Fall sein, wenn das Auslöseglied in seiner Position, in der das Sperrmittel an dem Anschlag anschlägt, verbleiben würde. Die Feder kann das Auslöseglied vorteilhaft in die proximale Richtung z.B. bis in die Ausgangsposition zurückbewegen, so dass das Sperrmittel durch das Zurückbewegen des Auslöseglieds aus der Sperrposition bewegt wird.

Mit anderen Worten ist es bevorzugt, dass die Abdeckkappe, insbesondere die Aktuatorfläche und/oder die Haltefläche, das Sperrmittel erst dann in seine Sperrposition auslenkt, wenn das Auslöseglied aus seiner Ausgangsposition in die proximale Richtung bewegt wird. Hierdurch wird sichergestellt, dass sich das Sperrmittel nur dann in die Sperrposition bewegt, wenn bei aufgesetzter Abdeckkappe das Auslöseglied aus seiner Ausgangsposition in die proximale Richtung bewegt wird, wie zum Beispiel bei einer Erschütterung, die zum Beispiel durch Herunterfallen des Autoinjektors auf den Boden verursacht werden könnte.

Zum Beispiel kann das Sperrmittel von der Aktuatorfläche in die Sperrposition bewegt werden und anschließend von der Haltefläche in der Sperrposition gehalten werden und/oder an der Haltefläche entlanggleiten, wenn das Auslöseglied gegen die Feder bzw. deren Federkraft aus der Ausgangsposition in die Position bewegt wird, in der das Sperrglied an dem Anschlag anschlägt. Insbesondere kann das Sperrmittel von der Haltefläche in der Sperrposition gehalten werden und/oder an der Haltefläche entlanggleiten und anschließend an der Aktuatorfläche entlanggleiten und dabei aus der Sperrposition federn, wenn das Auslöseglied von der Feder bzw. deren Federkraft aus seiner Position, in der das Sperrglied an dem Anschlag anschlägt, in die distale Richtung insbesondere in die Ausgangsposition bewegt wird.

Das Sperrmittel kann eine vorzugsweise in die proximale Richtung weisende Kontaktfläche aufweisen, welche an dem Anschlag anschlägt, wenn das Auslöseglied aus seiner Ausgangsposition in proximale Richtung bewegt wird und das Sperrmittel in seiner Sperrposition ist. Die Kontaktfläche kann zum Beispiel von dem freien Ende des vorzugsweise arm- oder zungenförmigen Sperrmittels gebildet werden, wobei das Sperrmittel zum Beispiel über die federnde Anlenkung einteilig mit dem Auslöseglied bzw. dessen Hauptkörper verbunden ist.

Das Sperrmittel kann zum Beispiel eine Nocke aufweisen, welche zum Beispiel von dem armoder zungenförmigen Sperrmittel zum Beispiel zu der Längsachse hin abragt. Das Sperrmittel kann an der Haltefläche anliegen und/oder an der Abdeckkappe oder der Haltefläche oder der Aktuatorfläche abgleiten, wodurch die Nocke und dadurch das Sperrmittel von der Abdeckkappe ausgelenkt werden. Zum Beispiel kann die Nocke von der Haltefläche oder der Aktuatorfläche ausgelenkt werden, insbesondere wenn das Auslöseglied in die proximale Richtung bewegt wird.

In besonders bevorzugten Ausführungen kann das Auslöseglied einen hülsenförmigen Abschnitt, welcher z. B. von dem Hauptkörper gebildet werden kann, aufweisen, wobei zwischen dem hülsenförmigen Abschnitt und dem Sperrmittel ein erster Spalt gebildet ist. Vorzugsweise umgibt der hülsenförmige Abschnitt das Sperrmittel. Das Gehäuse kann einen weiteren hülsenförmigen Abschnitt aufweisen, wobei zwischen dem hülsenförmigen Abschnitt des Gehäuses und dem Anschlag ein zweiter Spalt gebildet ist. Zum Beispiel kann der hülsenförmige Abschnitt des Gehäuses den Anschlag umgeben. Bevorzugt ist, dass der hülsenförmige Abschnitt des Auslöseglieds in den zweiten Spalt und/oder der Anschlag in den ersten Spalt bewegt werden, wenn das Auslöseglied aus der Ausgangsposition in die Auslöseposition bewegt wird. Hierdurch lässt sich z. B. eine besonders platzsparende Anordnung für einen der bekannten Autoinjektoren erzielen, ohne dass größere konstruktive Änderungen vorgenommen werden müssen.

Besonders bevorzugt kann die Nadel von einer an dem Produktbehälter befestigten Nadelabdeckkappe, die optional als Nadelschutzkappe bezeichnet werden kann, abgedeckt sein. Der Produktbehälter kann zum Beispiel distal des zylindrischen Abschnitts einen Bereich aufweisen, der die Nadel umgibt und fest mit der Nadel und dem zylindrischen Abschnitt des Produktbehälters verbunden ist. Die Nadelabdeckkappe kann an diesem Bereich befestigt sein. Allgemein kann die Nadel reib- und/oder formschlüssig an dem Produktbehälter, insbesondere an dem Bereich befestigt sein. Die Nadelabdeckkappe kann zum Beispiel eine Kappe aus einem elastischen Material, wie zum Beispiel Kautschuk oder Gummi sein. Alternativ kann die Nadelabdeckkappe aus einem festen, zum Beispiel hülsenförmigen Material gebildet sein, wobei solche Abdeckkappen als "rigid needle shield" bekannt sind. Die Abdeckkappe ist insbesondere so mit der davon separaten Nadelabdeckkappe gekoppelt, dass die Nadelabdeckkappe beim Abnehmen der Abdeckkappe von dem Gehäuse von dem Produktbehälter entfernt wird. Die Abdeckkappe kann hierfür ein Eingriffsglied aufweisen, welches an oder in die Nadelabdeckkappe an- oder eingreift, so dass die Nadelabdeckkappe mit abgezogen wird. Zum Beispiel kann das Eingriffsglied eine Kralle sein, die sich in der Nadelabdeckkappe verkrallt. Alternativ kann das Eingriffsglied ein hakenförmiges Element sein, welches zum Beispiel an dem proximalen Ende der Nadelabdeckkappe angreift, insbesondere in den Spalt zwischen der Nadelabdeckkappe und der Schulter, mit der sich der zylindrische Bereich des Spritzenkörpers verjüngt, eingreifen. Das hakenförmige Eingriffsglied zieht dann beim Entfernen der Abdeckkappe die Nadelabdeckkappe von dem Produktbehälter oder Injektionsnadel ab.

Im Folgenden wird eine besonders bevorzugte Ausführung anhand von Figuren beschrieben. Die dabei offenbarten Merkmale bilden die Erfindung einzeln und in jeder Merkmalskombination vorteilhaft weiter. Es zeigen:
- Figur 1: verschiedene Schnittdarstellungen eines distalen Abschnitts eines Autoinjektors mit an dem Gehäuse angeordneter Abdeckkappe,
- Figur 2: die Ansichten aus Figur 1, wobei ein Sperrmittel in seiner Sperrposition ist,
- Figur 3: die Ansichten aus Figur 1, wobei die Abdeckkappe entfernt ist und
- Figur 4: die Ansichten aus Figur 3, wobei ein Auslöseglied in seiner Auslöseposition ist.

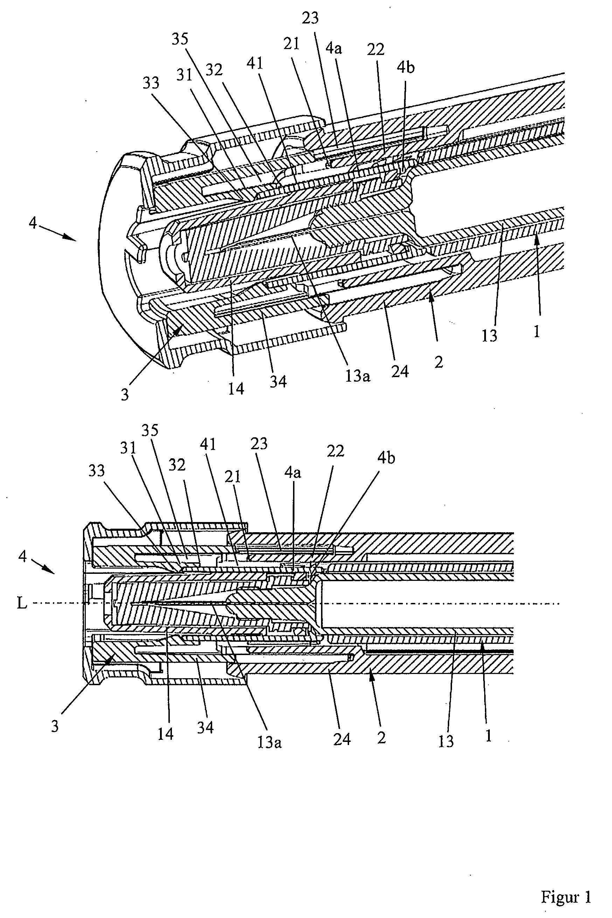

Der Autoinjektor weist ein hülsenförmiges, längliches Gehäuse 2 mit einer Längsachse L auf. In den Figuren 1 bis 4 wird lediglich der vordere bzw. distale Abschnitt des Autoinjektors dargestellt. Der im proximalen oder hinteren Teil untergebrachte Antriebsmechanismus ist dem zuständigen Fachmann geläufig und kann zum Beispiel so ausgestaltet sein, wie in der EP 2 742 962 A2 oder der WO 2008/113199 A1.

An dem distalen Ende des Autoinjektors ist in seinem Auslieferungszustand (Figur 1) eine Abdeckkappe 4 angeordnet, die an dem Gehäuse 2 zum Beispiel reib- und/oder formschlüssig befestigt ist und die vor der Verwendung des Autoinjektors abgezogen oder abgedreht, und entfernt wird.

In dem Gehäuse 2 ist ein Produktbehälter 13 in der Gestalt einer Spritze aufgenommen, wie zum Beispiel in Bezug auf das Gehäuse 2 entlang der Längsachse L verschiebbar oder unverschiebbar aufgenommen. Der Produktbehälter 13 weist einen hülsenförmigen Spritzenkörper auf, der einen Kolben, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 3 ist ein flüssiges Produkt, insbesondere Medikament, innerhalb des Spritzenkörpers angeordnet, wobei das flüssige Produkt durch Verschieben des Kolbens in eine Ausschüttrichtung, d. h. in die distale Richtung oder zu der Injektionsnadel 13a hin, durch die hohle Injektionsnadel 13a aus dem Produktbehälter 13 ausgeschüttet wird. Der Spritzenkörper weist an seinem proximalen Ende einen sogenannten Fingerflansch (nicht gezeigt) auf, der radial nach außen über den Außenumfang des zylindrischen Spritzenkörpers ragt.

Der Produktbehälter 13 ist in einem Produktbehälterhalter, der als Spritzenhalter 1 bezeichnet wird, so aufgenommen, dass er zumindest gegen eine Bewegung entlang der Längsachse L in distale Richtung relativ zu dem Spritzenhalter 1 gesichert ist. Der Spritzenhalter 1 kann mit dem Gehäuse 2 formschlüssig verbunden, insbesondere verrastet sein, oder relativ zu dem Gehäuse entlang der Längsachse L verschiebbar sein, je nach Ausführung des Autoinjektors. Sofern der Spritzenhalter 1 mit dem Gehäuse 2 verrastet wird, kann das Gehäuse 2 hierfür Ausnehmungen aufweisen, in die Rastelemente, die am Spritzenhalter 1 gebildet werden, eingreifen. Der Spritzenhalter 1 weist mindestens eine nach innen ragende Schulter auf, an der sich ein verjüngender Abschnitt des Produktbehälters 13, der distal des zylindrischen Splintzenkörpers angeordnet ist, abstützt.

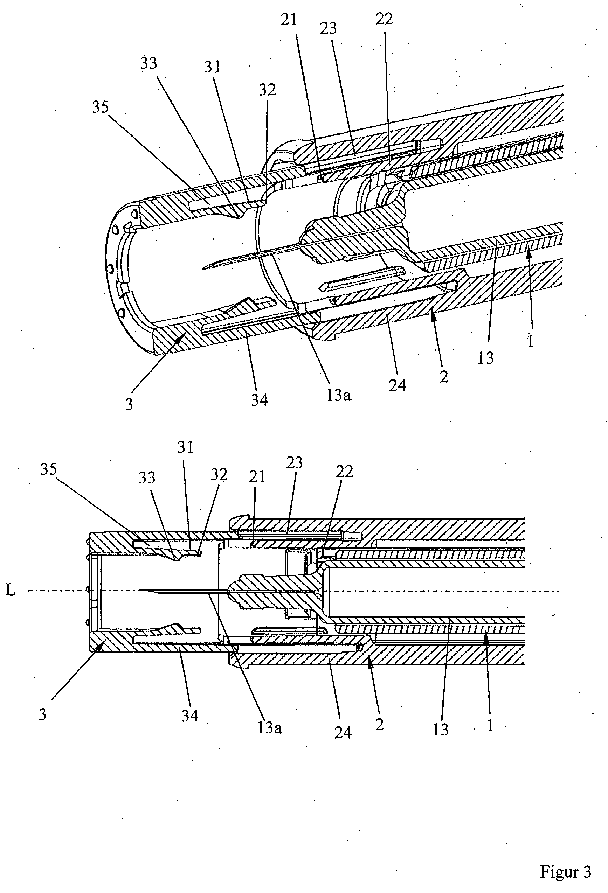

Der Autoinjektor weist an seinem distalen Ende ein hülsenförmiges Auslöseglied 3, insbesondere Nadelschutzhülse, auf, welches in seiner Ausgangsposition (Figuren 1 und 3) über das distale Ende des Gehäuses 2 ragt. Das Auslöseglied 3 wird bei aufgesetzter Abdeckkappe 4 vollständig von dieser umgeben. Das Auslöseglied 3 weist ein federnd angeordnetes Sperrmittel 31, welches zungen- oder armförmig ausgestaltet ist, auf. Das längliche Sperrmittel 31 erstreckt sich in etwa parallel zu der Längsachse L und weist mit seinem freien Ende in die proximale Richtung. Mit seinem anderen Ende ist es über einen federnden Abschnitt mit dem Auslöseglied 3, insbesondere einem hülsenförmigen Hauptkörper des Auslöseglieds 3, einstückig verbunden, insbesondere mit seinem distalen Ende. Das freie Ende bildet eine Kontaktfläche 32 für einen Anschlag 21, der von dem Gehäuse 2 gebildet wird. Die Kontaktfläche 32 weist in die proximale Richtung, wobei die Anschlagfläche 21 in die distale Richtung weist. Das Sperrmittel 31 weist eine Nocke 33 auf, die zu der Längsachse L hin abragt. Die Nocke 33 ist zwischen dem freien Ende und dem federnden Abschnitt des Sperrmittels 31 angeordnet. Das Auslöseglied 3 weist einen hülsenförmigen Abschnitt 34 auf, zwischen dem und dem Sperrmittel 31 ein erster Spalt 35 gebildet ist. Das Gehäuse 2 weist einen hülsenförmigen Abschnitt 24 auf, wobei zwischen dem hülsenförmigen Abschnitt 24 und dem Anschlag 21, insbesondere einen Gehäuseabschnitt 22, welcher den Anschlag 21 bildet, ein zweiter Spalt 23 gebildet ist.

Das Auslöseglied 3 ist relativ zu dem Gehäuse 2 in die proximale Richtung verschiebbar, wobei der hülsenförmige Abschnitt des Auslöseglieds 3 in den zweiten Spalt 23 und der Anschlag 21, insbesondere der Gehäuseabschnitt 22, in den ersten Spalt 35 verschoben werden oder verschiebbar sind.

Durch Verschieben des Auslöseglieds 3 in proximale Richtung bis in eine Auslöseposition kann die Produktausschüttung oder ein Einstechvorgang in bekannter Weise ausgelöst werden.

In Figur 1 befindet sich die Abdeckkappe 4 in ihrer an dem Gehäuse 2 befestigten Position. Die Abdeckkappe 4 bildet eine Haltefläche 41, die proximal der Nocke 33 angeordnet ist, wenn sich das Auslöseglied 3 in seiner Ausgangsposition befindet (Figur 1). Distal der Haltefläche 41 weist die Abdeckkappe 4 eine Ausnehmung für die Nocke 33 auf, so dass das Sperrmittel 31 durch seine federnde Anordnung sich in einer Freigabeposition oder Position außerhalb seiner Sperrposition befindet, wobei die Nocke 33 in dieser Ausnehmung angeordnet ist. Die Kontaktfläche 32 ist hierbei außerhalb einer axialen Flucht entlang der Längsachse L mit dem Anschlag 21.

Wenn der Autoinjektor zum Beispiel versehentlich auf den Boden fällt, kann unter Umständen das Auslöseglied 3 aufgrund seiner Massenträgheit relativ zu dem Gehäuse 2 in die proximale Richtung verschoben werden. Um zu verhindern, dass das Auslöseglied 3 dabei bis in seine Auslöseposition verschoben wird, was zur Folge hätte, dass die Produktausschüttung ungewollt ausgelöst wird, wird die Bewegung des Auslöseglieds 3 bis in seine Auslöseposition verhindert, indem das Sperrmittel 31, insbesondere die Kontaktfläche 32 an dem Anschlag 21 anschlägt.

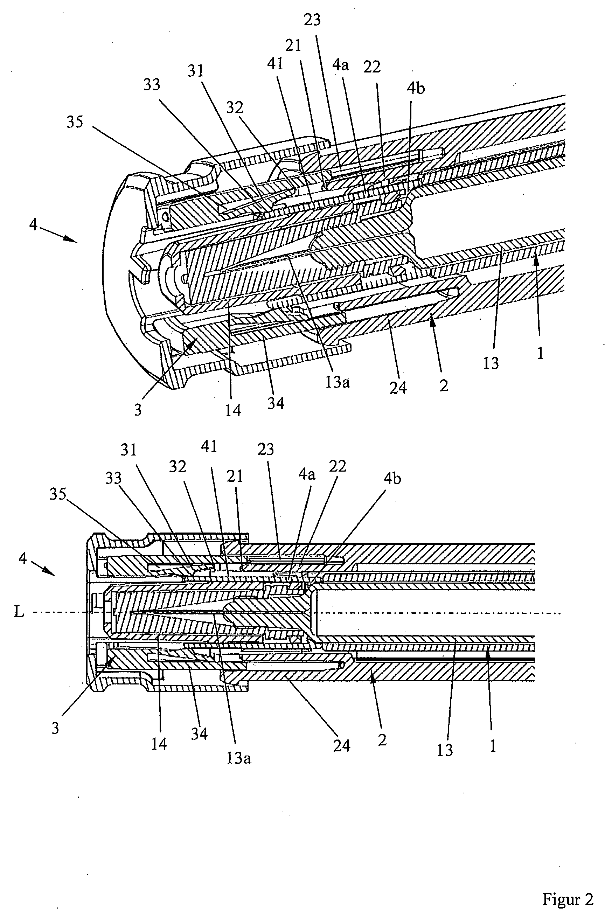

Wenn bei aufgesetzter Abdeckkappe 4 das Auslöseglied 3 aus seiner Ausgangsposition (Figur 1) relativ zu dem Gehäuse 2 in die proximale Richtung bewegt wird, wird das Sperrmittel 31 aus seiner Freigabeposition (Figur 1) in die Sperrposition (Figur 2) ausgelenkt. Die Abdeckkappe 4 weist hierzu eine Aktuator- oder Getriebefläche auf, an welche die Nocke 33, insbesondere eine abgeschrägte Getriebefläche der Nocke 33 abgleitet. Hierdurch wird die Nocke 33 und somit auch das Sperrmittel 31 federnd ausgelenkt, insbesondere von der Längsachse L weg. Die von der Längsachse L weg weisende, d. h. nach außen weisende Haltefläche 41, an der die Nocke 33 dann anliegt, hält das Sperrmittel 31 in seiner Sperrposition (Figur 2). In der Sperrposition befindet sich die Kontaktfläche 32 in axialer Flucht entlang der Längsachse L mit dem Anschlag 21. Hierdurch wird bewirkt, dass die Kontaktfläche 32 an dem Anschlag 21 anschlägt und somit eine Bewegung des Auslöseglieds 3 bis in seine Auslöseposition blockiert. Hierdurch wird vorteilhaft bewirkt, dass eine versehentliche Auslösung des Autoinjektors verhindert wird, solange die Abdeckkappe 4 an dem Gehäuse 2 befestigt ist.

Zur Vorbereitung der Verabreichung des Produkts wird die Abdeckkappe 4 von dem Gehäuse 2 entfernt. Durch das Entfernen der Abdeckkappe 4 wird gleichzeitig eine Nadelabdeckkappe 14, welche die Nadel 13a abdeckt, von dem Produktbehälter 13 entfernt. Im Ausgangs- oder Auslieferungszustand des Autoinjektors (Figur 1), d. h., wenn die Abdeckkappe 4 an dem Autoinjektor angeordnet ist, wird die Nadel 13a von der Nadelabdeckkappe 14, die in dem gezeigten Beispiel als sogenanntes und dem Fachmann als solches bekanntes "rigid needle shield", alternativ als "soft needle shield", ausgebildet ist, abdeckt, um die Nadel 13a vor Verschmutzung zu schützen bzw. die Nadel 13a und das Medikament steril zu halten. Die Nadelabdeckkappe 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter des Spritzenhalter ist zwischen dem Spritzenkörper und dem proximalen Ende der Nadelabdeckkappe 14 angeordnet, insbesondere so, dass zwischen der Nadelabdeckkappe 14 und der Schulter ein - wenngleich auch geringer - Spalt entsteht, um zu verhindern, dass die Schulter eine Kraft auf die Nadelabdeckkappe 14 ausübt, wodurch zum Beispiel die Sterilität der Nadel 13a oder des flüssigen Produkts gefährdet werden könnte. Die Abdeckkappe 4 ist mit dem Gehäuse 2 oder einer Nadelschutzhülse 3 lösbar verschnappt, wobei diese Verschnappung gelöst wird, wenn die Abdeckkappe 4 von dem Gehäuse 2 oder der Nadelschutzhülse 3 entfernt wird. Die Verschnappung kann zum Beispiel durch eine Schnappgeometrie gebildet werden. Die Abdeckkappe 4 weist insbesondere einen Schnapphaken 4a auf, der einen Schnapper 4b aufweist, der in eine Lücke zwischen dem Spritzenkörper, insbesondere dessen sich verjüngenden Bereich, und dem proximalen Ende der Nadelabdeckkappe 14 eingreift. Wenn die Abdeckkappe 4 von dem Autoinjektor entfernt wird, hakt der Schnapper 4b in das proximale Ende der Nadelabdeckkappe 14 ein, wodurch die Nadelabdeckkappe 14 von dem Produktbehälter 13 gelöst und zusammen mit der Abdeckkappe 4 von dem Autoinjektor entfernt wird (Figur 3).

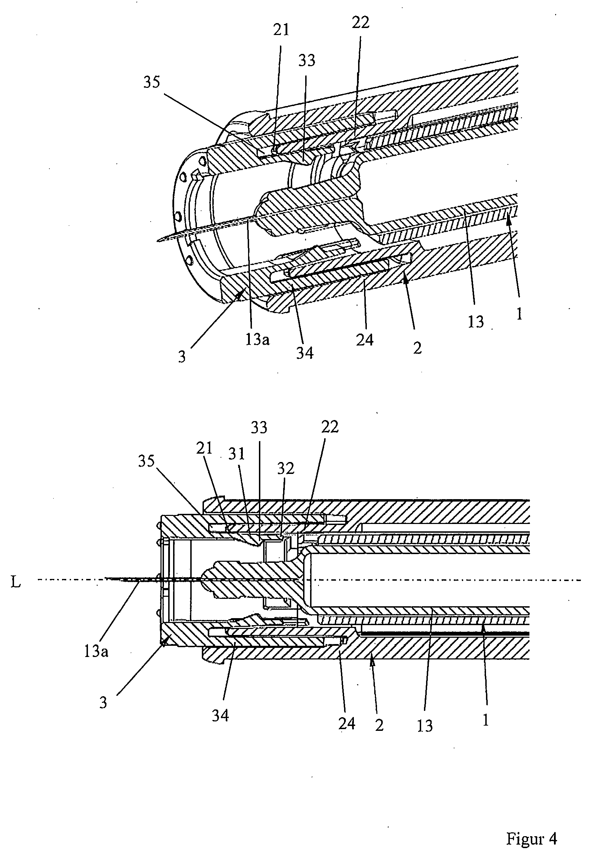

In Figur 3 wird der vordere Teil des Autoinjektors dargestellt, wobei die Abdeckkappe 4 zusammen mit der Nadelabdeckkappe 14 entfernt ist. Der Autoinjektor ist hierdurch für die Verabreichung vorbereitet. Für die Verabreichung wird das distale Ende des Auslöseglieds 3 an die gewünschte Einstichstelle des Patienten angesetzt. Das Gehäuse 2 wird vom Verwender zu der Einstichstelle hin gedrückt, wobei sich hierdurch das Auslöseglied 3 relativ zu dem Gehäuse 2 in die proximale Richtung bewegt. Dadurch, dass die Abdeckkappe 4 entfernt ist, wird das Sperrmittel 31 nicht mehr in die Sperrposition ausgelenkt. Das Sperrmittel 31 befindet sich in seiner Freigabeposition. Das Auslöseglied 3 kann somit bis in seine Auslöseposition verschoben werden (Figur 4), wobei die Kontaktfläche 32 an dem Anschlag 21 vorbei bewegt wird, da sich die Kontaktfläche 32 nicht in einer axialen Flucht entlang der Längsachse L mit dem Anschlag 21 befindet.

In der in Figur 4 gezeigten Auslöseposition steht die Injektionsnadel 13a über das distale Ende des Auslöseglieds 3 hervor, insbesondere mit einem Maß, welches der gewünschten Einstichtiefe entspricht. In der Ausgangsposition (Figur 3) wird die Injektionsnadel 13a von dem vorzugsweise hülsenförmigen Auslöseglied 3 umgeben. Insbesondere steht das Auslöseglied 3 distal über das distale Ende der Injektionsnadel 13a, wenn sich das Auslöseglied in seiner Ausgangsposition befindet.

Nach erfolgter Pröduktausschüttung kann eine zum Beispiel durch das Bewegen des Auslöseglieds 3 in die proximale Richtung gespannte Feder das Auslöseglied 3 relativ zu dem Gehäuse 2 in die distale Richtung verschieben, um eine Nadelschutzposition (nicht gezeigt) einzunehmen, in der das distale Ende des Auslöseglieds 3 über das distale Ende der Injektionsnadel 13a steht. In der Nadelschutzposition wird das Auslöseglied 3 vorzugsweise mit dem Gehäuse 2 so verrastet, dass es nicht mehr in die proximale Richtung verschiebbar ist, oder zumindest nicht mehr soweit verschiebbar ist, dass das distale Ende der Injektionsnadel 13a aus dem distalen Ende der Nadelschutzhülse 3 hervortritt.

### Bezugszeichenliste

- 1: Produktbehälterhalter / Spritzenhalter
- 13: Produktbehälter / Spritze
- 13a: Injektionsnadel
- 14: Nadelabdeckkappe

- 2: Gehäuse
- 21: Anschlag
- 22: Gehäuseabschnitt
- 23: zweiter Spalt
- 24: hülsenförmiger Abschnitt

- 3: Auslöseglied / Nadelschutzhülse
- 31: Sperrmittel
- 32: Kontaktfläche
- 33: Nocke
- 34: hülsenförmiger Abschnitt / Hauptkörper
- 35: erster Spalt

- 4: Abdeckkappe
- 4a: Schnapphaken
- 4b: Schnapper
- 41: Haltefläche

- L: Längsachse

## Patentansprüche

1. Autoinjektor zur Ausschüttung eines flüssigen Produkts, insbesondere Medikaments, umfassend:
a) ein Gehäuse (2) und einen in dem Gehäuse (2) angeordneten Produktbehälter (13), insbesondere Spritze, der einen verschiebbaren Kolben aufweist, wobei der Kolben mittels eines Vortriebsglieds zur Ausschüttung des in dem Produktbehälter (13) enthaltenen Produkts in eine distale Richtung verschiebbar ist,
b) eine Abdeckkappe (4), welche am distalen Ende des Gehäuses (2) angeordnet und von dem Gehäuse (2) abnehmbar ist,
c) ein insbesondere hülsenförmiges Auslöseglied (3), welches in seiner Ausgangsposition über das distale Ende des Gehäuses (2) ragt,
d) wobei das Auslöseglied (3) für die Auslösung der Ausschüttung relativ zu dem Gehäuse (2) aus der Ausgangsposition in eine proximale Richtung in eine Auslöseposition bewegbar ist, wenn die Abdeckkappe (4) von dem Gehäuse (2) abgenommen ist, und
e) wobei das Auslöseglied (3) gegen die Bewegung in die Auslöseposition blockiert ist, wenn die Abdeckkappe (4) am Gehäuse (2) angeordnet ist,
**dadurch gekennzeichnet, dass**
f) das Auslöseglied (3) ein Sperrmittel (31) aufweist, wobei das Sperrmittel (31) von der Abdeckkappe (4) in eine Sperrposition, in der das Sperrmittel (31) einem von dem Autoinjektor gebildeten Anschlag (21) gegenüberliegt, auslenkbar ist, wobei das Sperrmittel (31) in einer Freigabeposition, die das Sperrmittel (31) einnimmt, wenn die Abdeckkappe (4) abgenommen ist, an dem Anschlag (31) vorbeibewegbar ist.

2. Autoinjektor nach Anspruch 1, **dadurch gekennzeichnet, dass** das Sperrmittel (31) federnd angeordnet ist.

3. Autoinjektor nach Anspruch 1 oder 2, **dadurch gekennzeichnet, dass** das Gehäuse (2) den Anschlag (21) bildet.

4. Autoinjektor nach einem der Ansprüche 1 bis 3, **dadurch gekennzeichnet, dass** das Sperrmittel (31) von dem Anschlag (21) entlang der Längsachse (L) des Gehäuses (2) beabstandet ist, wenn das Auslöseglied (3) in seiner Ausgangsposition ist.

5. Autoinjektor nach einem der Ansprüche 1 bis 4, **dadurch gekennzeichnet, dass** die Abdeckkappe (4) eine Haltefläche (41) aufweist, an der das Sperrmittel (31) anliegt, wenn es in seiner Sperrposition ist.

6. Autoinjektor nach Anspruch 5, **dadurch gekennzeichnet, dass** die Haltefläche (41) so konfiguriert ist, dass sie das Sperrmittel (31) in der Sperrposition hält, wenn das Auslöseglied (3) in seiner Ausgangsposition ist.

7. Autoinjektor nach Anspruch 5, **dadurch gekennzeichnet, dass** die Haltefläche (41) so konfiguriert ist, dass das zusammen mit dem Auslöseglied (3) bewegbare Sperrmittel (31) erst an der Haltefläche (41) anliegt, wenn das Sperrmittel (31) zu dem Anschlag (21) hin und das Auslöseglied (3) aus der Ausgangsposition in die proximale Richtung verschoben wurden.

8. Autoinjektor nach einem der Ansprüche 5 und 7, **dadurch gekennzeichnet, dass** die Abdeckkappe (4) das Sperrmittel (31) in seine Sperrposition auslenkt, wenn das Auslöseglied (3) aus seiner Ausgangsposition in die proximale Richtung bewegt wird.

9. Autoinjektor nach einem der Ansprüche 1 bis 8, **dadurch gekennzeichnet, dass** das Sperrmittel (31) eine bevorzugt in die proximale Richtung weisende Kontaktfläche (32) aufweist, welche an dem Anschlag (21) anschlägt, wenn das Auslöseglied (3) aus seiner Ausgangsposition in die proximale Richtung bewegt wird.

10. Autoinjektor nach einem der Ansprüche 1 bis 9, **dadurch gekennzeichnet, dass** das Sperrmittel (31) eine Nocke (33) aufweist, welche an der Haltefläche (41) anliegt oder/und an der Abdeckkappe (4) abgleitet, wodurch die Nocke (33) und dadurch das Sperrmittel (31) von der Abdeckkappe (4) ausgelenkt werden.

11. Autoinjektor nach einem der Ansprüche 1 bis 10, **dadurch gekennzeichnet, dass** das Auslöseglied (3) einen hülsenförmigen Abschnitt (34) aufweist, wobei zwischen dem hülsenförmigen Abschnitt (34) und dem Sperrmittel (31) ein erster Spalt (35) gebildet ist, und dass das Gehäuse (2) einen hülsenförmigen Abschnitt (24) aufweist, wobei zwischen dem hülsenförmigen Abschnitt (24) und dem Anschlag (21) ein zweiter Spalt (23) gebildet ist, wobei der hülsenförmige Abschnitt (34) des Auslöseglieds (3) in den zweiten Spalt (23) und/oder der Anschlag (21) in den ersten Spalt (35) bewegt werden, wenn das Auslöseglieds (3) in die Auslöseposition bewegt wird.

12. Autoinjektor nach einem der Ansprüche 1 bis 11, **gekennzeichnet durch** eine Feder, wobei die Feder gespannt wird, wenn das Auslöseglied (3) aus seiner Ausgangsposition in die proximale Richtung bewegt wird, wobei das Auslöseglied (3) mittels der Feder in die distale Richtung verschiebbar ist.

13. Autoinjektor nach einem der Ansprüche 1 bis 12, **dadurch gekennzeichnet, dass** der Produktbehälter (13) eine Nadel (13a) aufweist, wobei das Auslöseglied (3) distal über das distale Ende der Nadel (13a) steht, wenn das Auslöseglied (3) in seiner Ausgangsposition ist und/oder wenn die Produktausschüttung erfolgt ist.

14. Autoinjektor nach einem der Ansprüche 1 bis 13, **dadurch gekennzeichnet, dass** der Produktbehälter (13) eine Nadel (13a) aufweist, wobei die Nadel (13a) von einer an dem Produktbehälter (13) befestigten Nadelabdeckkappe (14) abgedeckt ist, wobei die Abdeckkappe (4) so mit der davon separaten Nadelabdeckkappe (14) gekoppelt ist, dass die Nadelabdeckkappe (14) beim Abnehmen der Abdeckkappe (4) von dem Gehäuse (2) von dem Produktbehälter (13) und/oder der Nadel (13a) entfernt wird.

15. Autoinjektor nach einem der Ansprüche 1 bis 14, **gekennzeichnet durch** eine Antriebsfeder, die auf das Vortriebsglied wirkt und so stark vorgespannt ist, dass sie das Produkt aus dem Produktbehälter (13) **durch** Verschieben des Vortriebsglieds um einen Ausschütthub und des Kolbens ausschütten kann.

## Claims

1. An autoinjector for dispensing a fluid product, in particular a medicament, including:
a) a housing (2) and a product container (13), in particular a syringe, which is arranged in the housing (2) and which has a displaceable plunger, wherein the plunger is displaceable in a distal direction by means of a forward drive member for dispensing of the product contained in the product container (13),
b) a cover cap (4) which is arranged at the distal end of the housing (2) and is removable from the housing (2),
c) an in particular sleeve-shaped triggering member (4) which in its starting position projects beyond the distal end of the housing (2),
d) wherein for triggering the dispensing operation the triggering member (3) is moveable relative to the housing (2) from the starting position in a proximal direction into a triggering position when the cover cap (4) is removed from the housing (2), and
e) the triggering member (3) is blocked against movement into the triggering position when the cover cap (4) is arranged on the housing (2),
**characterised in that**
f) the triggering member (3) has a locking means (31), wherein the locking means (31) can be deflected by the cover cap (4) into a locking position in which the locking means (3) is opposite to an abutment (21) formed by the autoinjector, wherein the locking means (31) can be moved past the abutment (31) in an enable position which the locking means (31) assumes when the cover cap (4) is removed.

2. An autoinjector according to claim 1 **characterised in that** the locking means (31) is resiliently arranged.

3. An autoinjector according to claim 1 or 2 **characterised in that** the housing forms the abutment (21).

4. An autoinjector according to one of claims 1 to 3 **characterised in that** the locking means (31) is spaced from the abutment (21) along the longitudinal axis (L) of the housing (2) when the triggering member (3) is in its starting position.

5. An autoinjector according to one of claims 1 to 4 **characterised in that** the cover cap (4) has a holding surface (41) against which the locking means (31) bears when it is in its locking position.

6. An autoinjector according to claim 5 **characterised in that** the configuration of the holding surface (41) is such that it holds the locking means (31) in the locking position when the triggering member (3) is in its starting position.

7. An autoinjector according to claim 5 **characterised in that** the configuration of the holding surface (41) is such that the locking means (31) which is moveable together with the triggering member (3) bears against the holding surface (41) only when the locking means (31) has been moved towards the abutment (21) and the triggering member (3) has been moved out of the starting position in the proximal direction.

8. An autoinjector according to one of claims 5 and 7 **characterised in that** the cover cap (4) deflects the locking means (31) into its locking position when the triggering member (3) is moved out of its starting position in the proximal direction.

9. An autoinjector according to one of claims 1 to 8 **characterised in that** the locking means (31) has a contact surface (32) which preferably faces in the proximal direction and which bears against the abutment (21) when the triggering member (3) is moved out of its starting position in the proximal direction.

10. An autoinjector according to one of claims 1 to 9 **characterised in that** the locking means (31) has a cam (33) which bears against the holding surface (41) and/or slides against the cover cap (4) whereby the cam (33) and thereby the locking means (31) are deflected by the cover cap (4).

11. An autoinjector according to one of claims 1 to 10 **characterised in that** the triggering member (3) has a sleeve-shaped portion (34), wherein a first gap (35) is formed between the sleeve-shaped portion (34) and the locking means (31), and the housing (2) has a sleeve-shaped portion (24), wherein a second gap (23) is formed between the sleeve-shaped portion (24) and the abutment (21), wherein the sleeve-shaped portion (34) of the triggering member (3) is moved into the second gap (23) and/or the abutment (21) is moved into the first gap (35) when the triggering member (3) is moved into the triggering position.

12. An autoinjector according to one of claims 1 to 11 **characterised by** a spring, wherein the spring is stressed when the triggering member (3) is moved out of its starting position in the proximal direction, wherein the triggering member (3) is displaceable in the distal direction by means of the spring.

13. An autoinjector according to one of claims 1 to 12 **characterised in that** the product container (13) has a needle (13a), wherein the triggering member (3) projects distally beyond the distal end of the needle (13a) when the triggering member (3) is in its starting position and/or when product dispensing is effected.

14. An autoinjector according to one of claims 1 to 13 **characterised in that** the product container (13) has a needle (13a), wherein the needle (13a) is covered by a needle cover cap (14) fixed to the product container (13), wherein the cover cap (4) is so coupled to the needle cover cap (14) which is separate therefrom that the needle cover cap (14) is moved away from the product container (13) and/or the needle (13a) when the cover cap (4) is removed.

15. An autoinjector according to one of claims 1 to 14 **characterised by** a drive spring which acts on the forward drive member and which is so greatly prestressed that it can dispense the product from the product container (13) by displacement of the forward drive member by a dispensing stroke and of the plunger.

## Revendications

1. Injecteur automatique pour le versement d'un produit liquide, en particulier d'un médicament, comprenant :
a) un boîtier (2) et un récipient de produit (13) agencé dans le boîtier (2), en particulier une seringue, qui présente un piston mobile, le piston étant mobile à l'aide d'un organe d'avancement pour le versement du produit contenu dans le récipient de produit (13) dans une direction distale,
b) un capuchon (4) qui est agencé à l'extrémité distale du boîtier (2) et peut être retiré du boîtier (2),
c) un organe de déclenchement (3) en particulier en forme de douille qui dépasse dans sa position de départ de l'extrémité distale du boîtier (2),
d) l'organe de déclenchement (3) pouvant être déplacé pour le déclenchement du versement par rapport au boîtier (2) depuis la position de départ dans une direction proximale vers une position de déclenchement lorsque le capuchon (4) est retiré du boîtier (2), et
e) l'organe de déclenchement (3) est bloqué pour le mouvement vers la position de déclenchement si le capuchon (4) est agencé sur le boîtier (2),
**caractérisé en ce que**
f) l'organe de déclenchement (3) présente un moyen de verrouillage (31), le moyen de verrouillage (31) pouvant être dévié du capuchon (4) dans une position de verrouillage, dans laquelle le moyen de verrouillage (31) fait face à une butée (21) formée par l'injecteur automatique, le moyen de verrouillage (31) pouvant passer devant la butée (31) dans une position de libération qu'occupe le moyen de verrouillage (31) lorsque le capuchon (4) est retiré.

2. Injecteur automatique selon la revendication 1, **caractérisé en ce que** le moyen de verrouillage (31) agencé sur ressort.

3. Injecteur automatique selon la revendication 1 ou 2, **caractérisé en ce que** le boîtier (2) forme la butée (21).

4. Injecteur automatique selon l'une quelconque des revendications 1 à 3, **caractérisé en ce que** le moyen de verrouillage (31) est espacé de la butée (21) le long de l'axe longitudinal (L) du boîtier (2) lorsque l'organe de déclenchement (3) est dans sa position de départ.

5. Injecteur automatique selon l'une quelconque des revendications 1 à 4, **caractérisé en ce que** le capuchon (4) présente une surface de retenue (41), contre laquelle repose le moyen de verrouillage (31) lorsqu'il est dans sa position de verrouillage.

6. Injecteur automatique selon la revendication 5, **caractérisé en ce que** la surface de retenue (41) est configurée de sorte qu'elle maintienne le moyen de verrouillage (31) dans la position de verrouillage lorsque l'organe de déclenchement (3) est dans sa position de départ.

7. Injecteur automatique selon la revendication 5, **caractérisé en ce que** la surface de retenue (41) est configurée de sorte que le moyen de verrouillage (31) mobile conjointement avec l'organe de déclenchement (3) ne repose contre la surface de retenue (41) que si le moyen de verrouillage (31) a été déplacé vers la butée (21) et l'organe de déclenchement (3) de la position de départ à la direction proximale.

8. Injecteur automatique selon l'une quelconque des revendications 5 et 7, **caractérisé en ce que** le capuchon (4) dévie le moyen de verrouillage (31) dans sa position de verrouillage lorsque l'organe de déclenchement (3) est déplacé de sa position de départ dans la direction proximale.

9. Injecteur automatique selon l'une quelconque des revendications 1 à 8, **caractérisé en ce que** le moyen de verrouillage (31) présente une surface de contact (32) tournée de préférence dans la direction proximale qui bute contre la butée (21) lorsque l'organe de déclenchement (3) est déplacé de sa position de départ dans la direction proximale.

10. Injecteur automatique selon l'une quelconque des revendications 1 à 9, **caractérisé en ce que** le moyen de verrouillage (31) présente une came (33) qui repose contre la surface de retenue (41) et/ou glisse sur le capuchon (4), par quoi la came (33) et par là même le moyen de verrouillage (31) sont déviés du capuchon (4).

11. Injecteur automatique selon l'une quelconque des revendications 1 à 10, **caractérisé en ce que** l'organe de déclenchement (3) présente une section en forme de douille (34), une première fente (35) étant formée entre la section en forme de douille (34) et le moyen de verrouillage (31), et **en ce que** le boîtier (2) présente une section en forme de douille (24), une seconde fente (23) étant formée entre la section en forme de douille (24) et la butée (21), la section en forme de douille (34) de l'organe de déclenchement (3) étant déplacée dans la seconde fente (23) et/ou la butée (21) dans la première fente (35) lorsque l'organe de déclenchement (3) est déplacé dans la position de déclenchement.

12. Injecteur automatique selon l'une quelconque des revendications 1 à 11, **caractérisé par** un ressort, le ressort étant tendu lorsque l'organe de déclenchement (3) est déplacé de sa position de départ à la direction proximale, l'organe de déclenchement (3) étant mobile à l'aide du ressort vers la direction distale.

13. Injecteur automatique selon l'une quelconque des revendications 1 à 12, **caractérisé en ce que** le récipient de produit (13) présente une aiguille (13a), l'organe de déclenchement (3) se trouvant distalement au-dessus de l'extrémité distale de l'aiguille (13a) lorsque l'organe de déclenchement (3) est dans sa position de départ et/ou lorsque le versement de produit est effectué.

14. Injecteur automatique selon l'une quelconque des revendications 1 à 13, **caractérisé en ce que** le récipient de produit (13) présente une aiguille (13a), l'aiguille (13a) étant recouverte d'un capuchon d'aiguille (14) fixé sur le récipient de produit (13), le capuchon (4) étant couplé avec le capuchon d'aiguille (14) séparé de celui-ci de sorte que le capuchon d'aiguille (14) soit retiré du récipient de produit (13) et/ou de l'aiguille (13a) lors du retrait du capuchon (4) du boîtier (2).

15. Injecteur automatique selon l'une quelconque des revendications 1 à 14, **caractérisé par** un ressort d'entraînement qui agit sur l'organe d'avancement et est précontraint si fortement qu'il peut verser le produit hors du récipient de produit (13) par déplacement de l'organe d'avancement d'une course de versement et du piston.
